# EUROPEAN PATENT APPLICATION

(11) **EP 3 546 947 A2**
(43) Date of publication of application: **02.10.2019**
(21) Application number: 19158484.6
(22) Date of filing: 08.07.2016
(51) Int. Cl.: G01N 33/68

(54) **BIOMARKER PANELS FOR BRAIN INJURY COMPLICATIONS**

(30) Priority: 13.07.2015 EP 15002074
(62) Divisional of application: 16738671.3
(71) Applicant: University of Geneva, 1211 Geneva (CH); Fundacio Hospital Universitary Vall d'Hebron, 08035 Barcelona (ES)
(72) Inventor: SANCHEZ, Jean-Charles, 1208 Genève (CH); MONTANER, Joan, 08037 Barcelona (ES)
(74) Representative: Helbig, Christian

(57) **Abstract**

The embodiments of the invention relate to the novel use of biomarkers, combinations of biomarkers, methods of screening, methods of screening using said biomarkers and biomarker combinations, kits, assay devices and algorithms.

## Description

The embodiments of the invention relate to the novel use of biomarkers, combinations of biomarkers, methods of screening, methods of screening using said biomarkers and biomarker combinations, kits, assay devices and algorithms.

### Background

Brain injury complications like infections occur in a high percentage of patients. Such complications are known and the aim in brain injury treatment is to minimize or treat such complications very early in order to improve the overall state and outcome of a patient.

Biomarkers are meanwhile known in the art for a number of uses e.g. the prediction for diseases and disorders or to stratify a patient group.

One problem in brain injury are complications like infections which have a negative impact on the patient and negative implications for the recovery of a patient. Accordingly, such complications are currently either treated with precautionary measures like unspecific antibiotics treatment in the case of infection with all their negative implications or only once such complications are obvious which implies a belated treatment implying also suboptimal patient care.

Accordingly, there is a long felt need to improve the treatment of brain injury complications. Another long felt need is the early identification of such brain injury complications in order to provide the patient with the appropriate treatment as early as possible. Moreover, there is a need to avoid unneeded and unnecessary or unspecific treatments.

Hence, an object underlying the present application is to provide for means to improve the treatment of brain injury complications, or to reduce the disadvantages of the prior art or to avoid them substantially.

Another object is to provide for the early identification of brain injury complications.

### Summary of Embodiments

In one aspect is disclosed a method for screening for a brain injury complications in a disease or disorder in a sample using markers.

In another aspect is disclosed a composition comprising or consisting of at least one marker or more useful for the detection of a brain injury complication.

In yet another aspect is disclosed a kit comprising or consisting of two or more markers.

In yet another aspect is disclosed an assay device using biomarkers for the detection or prediction for brain injury complication.

In yet another aspect is disclosed a method for stratifying individuals for the treatment of brain injury complications.

In yet another aspect is disclosed a method for the prediction of the susceptibility of an individual to develop a brain injury complication.

In yet another aspect is disclosed an algorithm useful in the analysis of brain injury complications.

### Brief Description of the Figures and Tables

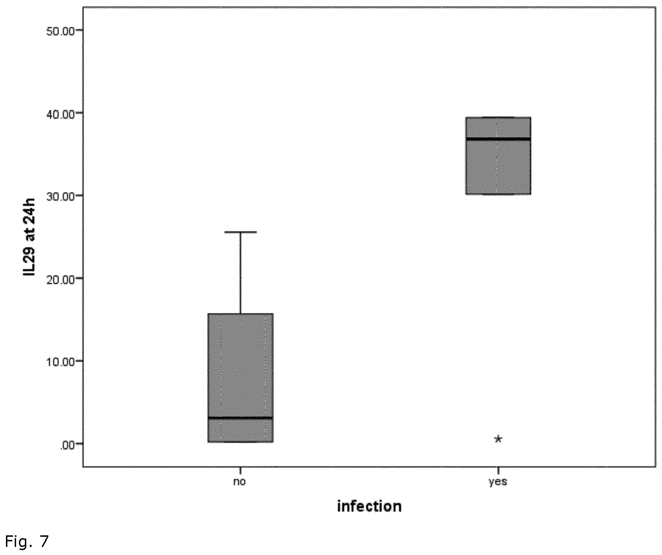

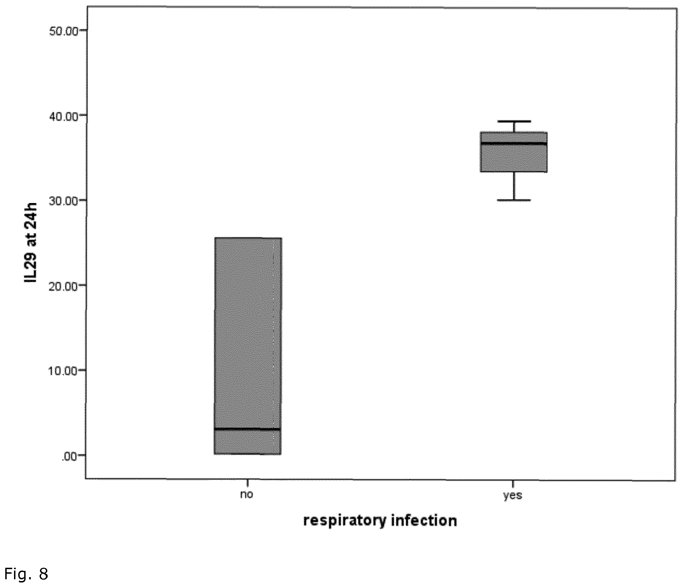

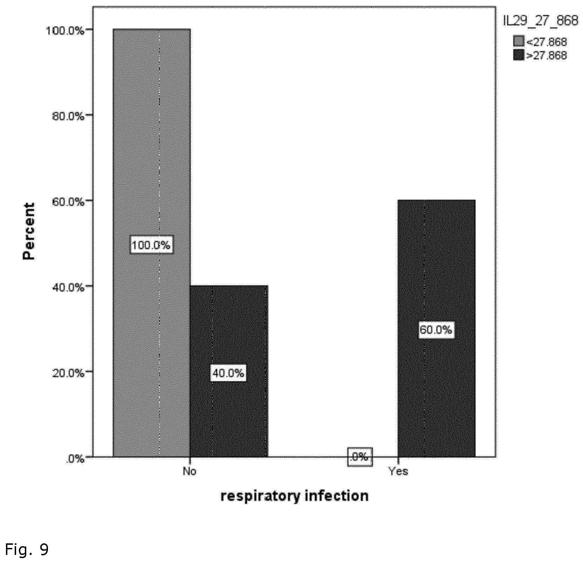

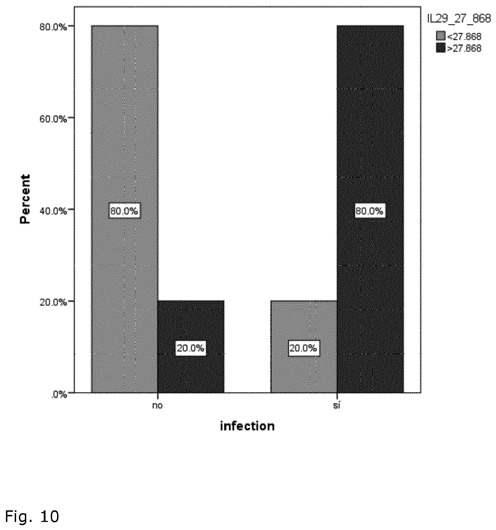

**Table 1** shows the performance of the different parameters as a single variable to detect infected and non-infected patients with SAA as the best blood molecule with an AUC of 75% (95% SE - 40% SP).
**Table 2** shows the performance of the best combination of two parameters with an AUC of 80% (100% SE - 61% SP).
**Table 3** shows the performance of the best combination of three parameters with an AUC of 89% (100% SE - 78% SP).
**Table 4** shows the performance of the best combination of four parameters with an AUC of 94% (100% SE - 83% SP).

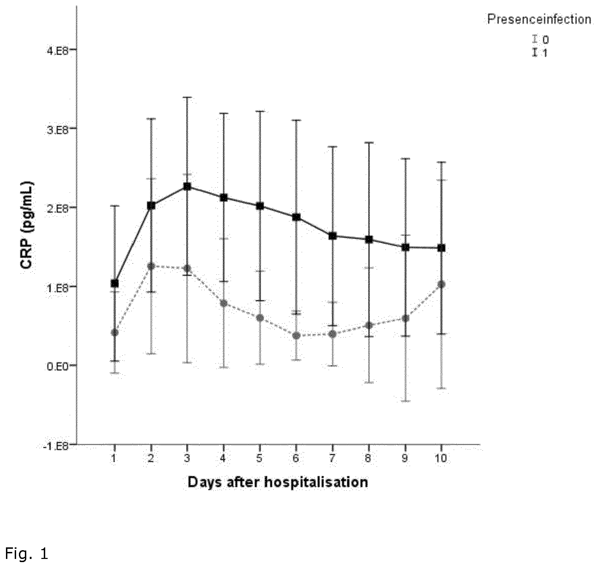

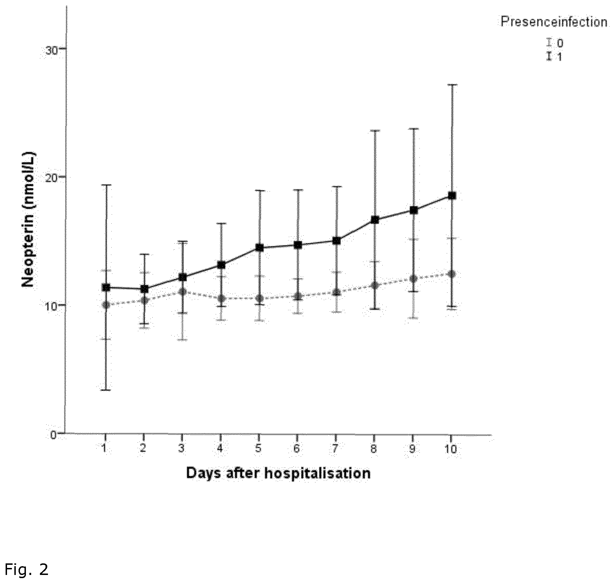

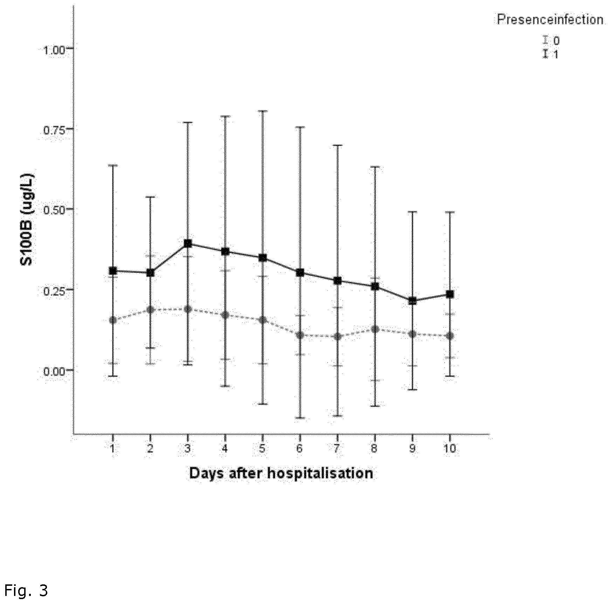

**Figures 1 to 4** show the kinetic during 10 days of SAA, CRP, Neopterin, and S100b with infected patients in green and non-infected patients in blue.

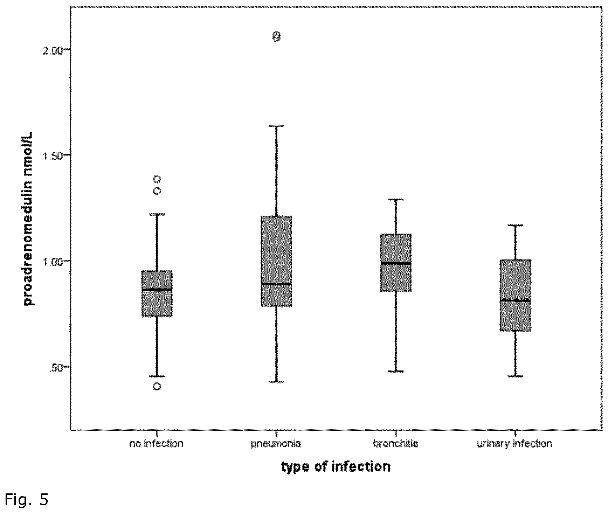

**Fig. 5** refers to Example 2 and shows different types of infections and a control.

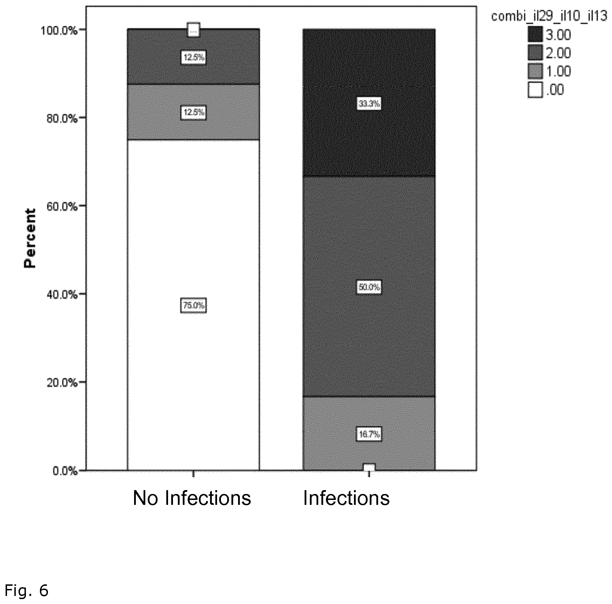

**Fig. 6** refers to Example 2; in 14 patients in whom we had also measured IL-10 and IL-13 , we may show that the combination of the three markers improves IL-29 accuracy. As shown in Figure when the three biomarkers are "positive" (darker bar part of the figure) we might identify correctly 33% of infected patients and when the three biomarkers are "negative" (whiter bar part of the figure) we might identify correctly 75% non infected patients (p=0.031).
   Cuttoffs identified to predict infection:
   - IL10 <1.536
   - IL13 <1.94
   - IL29 > 27.868
**Fig. 7 to 10** refer to Example 3 and the relevance of IL-29 as biomarker.
**Fig. 11****:** depicts the expression of SAA in depleted plasma samples of the 14 most abundant proteins analysed using MS of infected (n = 3) and non-infected patients (n = 3) using a proteomics strategy. Significance level is reported by * (p < 0.05).
**Fig. 12****:** refers to Example 4 and shows the kinetics of SAA concentrations measured using ELISA from hospital admission up to five days later in the StrokeChip (left) and COSMOS (right) cohorts. Comparison between the two groups was made using the Mann-Whitney U test. Significance level is reported after the Bonferroni correction (p < 0.01).

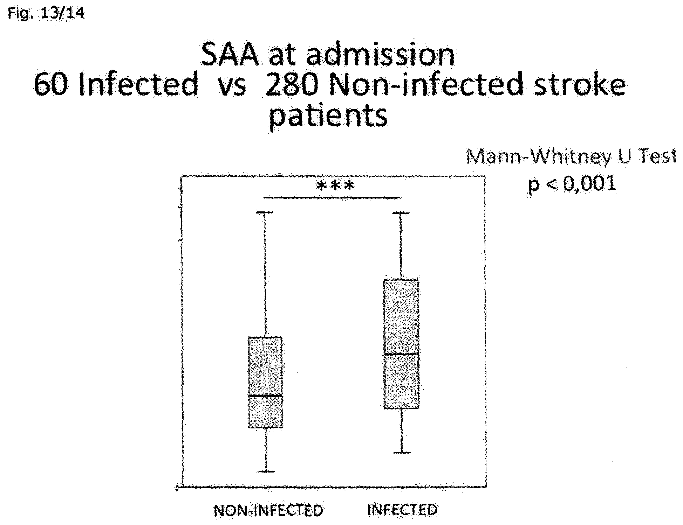

**Fig. 13** shows SAA at admission: 60 infected versus 280 non-infected stroke patients

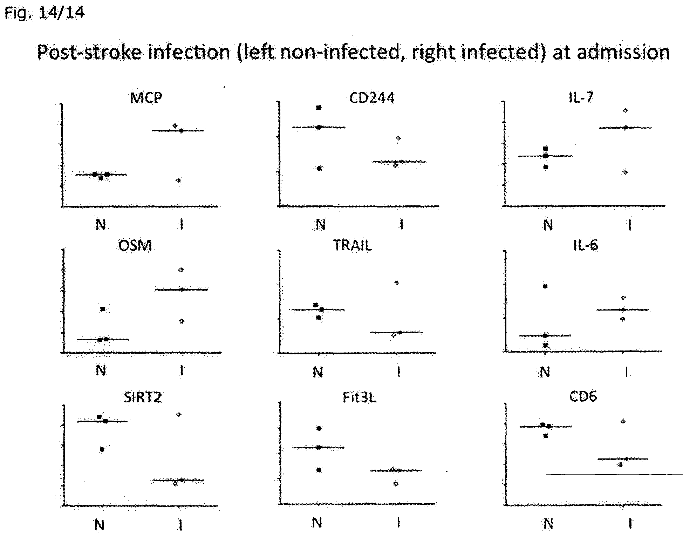

**Fig. 14** ((Figure 4)): Verification of MCP-3, CD244, Il-7, OSM, TRAIL, IL-6, SIRT2, Fit3L and CD6 concentrations as measured *using ELISA (Olink) at hospital* at admission (left, non-infected n=3; right, future infected n=3).

### Detailed Description

In the following certain terms of the invention will be defined in more detail.

"Brain injury" in the sense of the disclosure is any state of a patient or individual which is the cause of sudden impact on the head or the individual. A particular brain injury is TBI or mTBI.

"Brain injury complication" in the sense of the disclosure is e.g. an infection, e.g. pneumonia, urinary tract infection, vasospasm and depression.

A "disease" or "disorder" in the sense of the disclosure is e.g. stroke, traumatic brain injury (TBI), or subarachnoid hemorrhage (SAH).

"TBI" in the sense of the invention is any brain injury caused by a traumatic incident as described above with reference to the prior art.

"Identification" or "identify" or "classify" in the sense of the disclosure is the analysis of a sample of an individual to assess whether the individual has a brain injury complication or is prone therefor.

"Diagnostic method" or "diagnostic" in the sense of the disclosure is any useful method with a suitable sequence of method steps for the detection, visualization and/or quantification of the test result generally known in the art.

"Assay" in the sense of the disclosure is any method generally known in the art to detect a disease or disorder or a brain injury complication like ELISA or any other standard methods for detection of biomarkers.

"Device" in the sense of the disclosure is a combination of the biomarkers or panel of biomarkers according to the disclosure that can be used to perform an assay for disease or disorder or brain injury complication detection. Examples are carrier plates, test stripes, biochip arrays or the like known in the art.

"Marker" or "biomarker" or "molecular marker" or "molecular biomarker" in the sense of the disclosure is any useful biomarker to detect in a sample of preferably blood, plasma, saliva, tears, CSF or urine a brain injury complication as disclosed herein or other disorders as described below; preferably the combination of at least two markers is suitable; the markers are used in a suitable assay setup wherein preferably the sensitivity is set to 100% and the specificity is preferably more than 40%, even more preferred more than 50%, more than 55%, more than 58%, 60% or 70%.

Preferred markers as disclosed are:
S100b, CRP1, Serum Amyloid A (SAA), SAA1, NP1, WBC, IP-10, IFN-γ, copeptin, procalcitonin, IL-6, IL-8, IL-13, IL-29, TNF-α, peroxiredoxin 1 to 6 family.

In addition to the "markers" as described above it is also within the scope of preferred embodiments that one, two, three or even more markers can be combined and in addition or in the alternative defining the patient or individual by age or GCS score. Age and GCS can thus be denoted as "marker" in the sense of the disclosure. Such markers like age and GCS can also be used in the sense of the disclosure to define a patient subgroup or subgroup of individuals. A preferred age group is below 50, 60, 70 or more than 50, 60, 65, 70 years of age. A GCS of 13 to 15 can preferably be used to define a patient subgroup and can be used in combination with any of the other markers defined herein. In such a manner the individuals can be stratified and patient groups can be formed both to adapt and increase the test performance or to reduce the markers needed to achieve a reliable test result and preferably to adjust the features of the detection method or the components of a test kit.

A marker "panel" in the sense of the disclosure is a combination of at least two biomarkers, preferably two or three markers, used in combination in a suitable setup or device.

"Sensitivity" in the sense of the disclosure refers to the assay result of true positives in the analysis of brain injury complication. Preferably the sensitivity in the analysis according to the disclosure is set to 80% and preferably higher, e.g. 85%, 90%, 95%, 100%. (i.e. no false negative diagnoses).

"Specificity" in the sense of the disclosure is the so-called true negative rate in an assay. The specificity is preferably targeted to be at least 80% and preferably higher, e.g. 85%, 90%, 95%, 100%.

A "sample" or "specimen" in the sense of the disclosure is any fluid or tissue useful for performing an assay or detection method to identify TBI, preferably mTBI. Preferably the sample is a blood, plasma, tears, saliva, CSF or urine sample taken from an individual. The sample is treated according to generally known procedures to keep or make them feasible for the marker analysis according to the invention.

In the following preferred embodiments of the invention will be described.

The invention relates to a method for screening for a brain injury complications in a disease or disorder in a sample comprising the steps of using a sample under suitable conditions and detecting at least one or more biomarkers, preferably blood biomarkers, under suitable conditions wherein the markers are selected from the group consisting of S100b, CRP1, Serum Amyloid A (SAA), SAA1, NP1, WBC, IP-10, IFN-γ, copeptin, procalcitonin, IL-6, IL-8, IL-10, IL-13, IL-29, TNF-α, IGFBP3, pro-adrenomedullin, sTREM-1, D-Dimer, presepsin (sCD14), peroxiredoxin 1 to 6, suPAR, Neopterin, MCP-3, CD244, Il-7, OSM, TRAIL, IL-6, SIRT2, Fit3L and CD6 or fragments, variants or mutants thereof.

The invention provides the unexpected advantage that a brain injury and the particular medical complications as described herein can be identified and screened for not only in a fully equipped hospital but by use of a simple test device anywhere and without the use of qualified medical personnel.

In a preferred embodiment a method is disclosed wherein the brain injury complication is selected from the group consisting of infection, e.g. pneumonia, urinary tract infection, vasospasm and depression, and the disease or disorder is selected from the group consisting of stroke, traumatic brain injury (TBI), subarachnoid hemorrhage (SAH)
In a preferred embodiment one or more biomarkers as disclosed herein are combined with a marker which is age, temperature, a defined GCS, GOS, NIHSS, A2DS2, Rankin and modified Rankin, Barthel Index, Fisher Score, or WFNS.

In a preferred method one or more biomarkers as disclosed above is used for screening a defined age or age group, temperature or a defined GCS, GOS, NIHSS, A2DS2, Rankin and modified Rankin, Barthel Index, Fisher Score, or WFNS.

In a preferred method the age group has an age of less than 50, less than 60, or more than 60, more than 70, or more than 80.

In the method as disclosed the sample can be taken from or be any fluid or tissue of an individual, preferably the sample is blood, plasma, urine, saliva, tears (lachrymal fluid), or CSF.

In another aspect the invention relates to a composition comprising or consisting of at least one marker or more useful for the detection of a brain injury complication of the group consisting of infection, e.g. pneumonia, hemorrhage, depression and vasospasm in a sample of an individual wherein the markers are selected from the group consisting of S100b, CRP1, Serum Amyloid A (SAA), SAA1, NP1, WBC, IP-10, IFN-γ, copeptin, procalcitonin, IL-6, IL-8, IL-10, IL-13, IL-29, TNF-α, IGFBP3, pro-adrenomedullin, sTREM-1, D-Dimer, presepsin (sCD14), peroxiredoxin 1 to 6, suPAR, Neopterin, MCP-3, CD244, Il-7, OSM, TRAIL, IL-6, SIRT2, Fit3L and CD6 or fragments, variants or mutants thereof.

Said composition advantageously is comprising or consisting of one, two or three markers wherein the markers are selected from S100b, CRP1, Serum Amyloid A (SAA), SAA1, NP1, WBC, IP-10, IFN-γ, copeptin, procalcitonin, IL-6, IL-8, IL-10, IL-13, IL-29, TNF-α, IGFBP3, pro-adrenomedullin, sTREM-1, D-Dimer, presepsin (sCD14), peroxiredoxin 1 to 6 family, suPAR, Neopterin or fragments, variants or mutants thereof. Preferred are combinations of SAA1, NP1, S100B1, CRP1 which comprise two or three of said markers, preferably with a particular age group as described above.

The disclosure encompasses also further subgroups of marker combinations being advantageous in terms of the test results that can be achieved. These subgroups are a combination of two, three, four or five markers selected from the group consisting S100b, CRP1, Serum Amyloid A (SAA), SAA1, NP1, WBC, IP-10, IFN-γ, copeptin, procalcitonin, IL-6, IL-8, IL-13, IL-29, TNF-α, peroxiredoxin 1 to 6 family or fragments, variants or mutants thereof and/or age below 50, 60, 70 or more than 50, 60, 65, 70 years of age and /or a GCS of 13 to 15, and/or WFNS and Fisher.

Other preferred subgroups of markers are pro-adrenomedullin, IL-10, SAA, IGFBP3 and IL-29, whereby preferred are at baseline pro-adrenomedullin, IL-10, SAA, IGFBP3, and at 24 hrs is IL-29, sTREM-1, D-Dimer, preseptin (sCD14), IL-13, suPAR and/or Neopterin wherein any combination of said markers is preferred and may lead to advantageous results.

Particularly preferred marker combinations are also apparent from the Tables disclosed below.

In another aspect the invention relates to a kit comprising or consisting of one or more markers as described herein.

In another aspect the invention relates to an assay device comprising or consisting of one or more markers as described herein.

Known assay setups and components may be used as is known in the art. It is preferred that the assay device comprises or consists of a biochip, biomarker panel, a carrier, or a test strip.

The assay and method as disclosed will be setup and designed in order to arrive at positive sensitivity and specificity as is required for the particular purpose and in combination with single components. It is preferred that the sensitivity is more than 80%, preferably 100%, and/or the specificity is more than 80%, preferably 100%.

In another aspect the disclosure relates to an algorithm and a method as disclosed herein making use of an algorithm.

As one aspect a method to identify a brain injury complication is described. The method and assays as disclosed herein can also be applied and useful for a method for stratifying individuals for the treatment of brain injury complications. Preferably it is within the disclosure of using a method, a composition, a kit, an assay, an assay device or/and an algorithm as described herein for stratifying individuals for a certain treatment.

Finally, the disclosure relates to a method for the prediction of the susceptibility of an individual to develop a brain injury complication using a method, a composition, a kit, an assay, an assay device or/and an algorithm as described herein.

As will be apparent from the experimental part that the subject matter described has the advantage that it achieves very reliable test results. Accordingly in preferred embodiments it provides for a method, composition, kit or assay wherein the sensitivity or specificity is more than 95%, preferably 100%, and the specificity or sensitivity is more than 50%, preferably more than 55%, more preferably more than 60%, and more preferably more than 70%.

In an alternative embodiment the disclosure provides for a method of or a system for analyzing in a specimen a medical condition wherein a medical device as described herein is applied under appropriate conditions, making use of any of the biomarkers described herein for the analysis of any of the disorders or diseases of the invention and making use of an algorithm wherein the test results are further defined by way of the algorithm, e.g. quantified.

The steps of such a method or system are in a preferred embodiment as follows:
The method and system are capable to analyze at least two test results in a sample of an individual, useful for the diagnosis of a medical condition like brain injury, with the system comprising:
(a) at least two databases comprising:
   (i) a first test result collected from a first diagnostic test;
   (ii) a second test result collected from a second diagnostic test different than the first diagnostic test;
   (iii) optionally, subsequent test result(s) collected from subsequent diagnostic test(s) different from the previous diagnostic test(s)
   (iv) optionally , secondary subject observations or measurements;
   (v) one or more diagnostic cut-offs associated with the first diagnostic test, with the second diagnostic test, with subsequent diagnostic tests, and with the subject observations or measurements, wherein such cut-offs collectively integrate to assess probability of brain injury status.
(b) one or more processors operatively encoded to automatically:
   (i) apply an interpretation algorithm to generate a subject result coordinate based on the database of test results.
   (ii) optionally apply a second interpretation algorithm to generate a probability of error in the subject result coordinate.

The following examples are intended to illustrate preferred aspects of the invention and are not meant to be construed as being restrictive for the invention or any aspect thereof.

### Examples

### Example 1

An example wherein the invention is useful is stroke patients. Stroke patients can serve as exemplary disease wherein the idea of the invention is applicable.

Stroke is, according to the WHO the leading cause for severe morbidity (first cause of motor handicap, second cause of dementia) and the second cause of overall mortality worldwide. In Switzerland stroke incidence has been estimated at 150/100'000 inhabitants¹. Infection is the most common complication during the first 30 days after ischemic stroke (IS) and even in specialist stroke units, stroke associated infections remain one of the major complications with frequencies between 21-65%²⁻³. In particular pneumonia is the most relevant stroke-associated infection⁴. It is associated with poor functional recovery (modified Rankin Scale score 3-6), with an odds ratio (OR) of 4.4 (95% CI 2.2-9.0)⁵. Moreover it is found to be an independent prognostic factor and major cause of death (OR 2.2, 95% CI 1.8-2.7)⁶⁻⁷ besides age and stroke severity (measured with the National Institutes of Health Stroke Scale (NIHSS))⁹. In the US, mean adjusted costs of hospitalization for patients with stroke associated pneumonia were $21,000 compared to $6,200 for stroke patients without pneumonia. In addition, patients with pneumonia were over 70% more likely to be discharged with requirements for extended care. Extrapolated to all patients hospitalized for stroke in the United States, the annual costs of stroke associated pneumonia is approximately $459 million¹⁰. Therefore, early initiation of antibiotic treatments is recommended if infection is present^{8, 11}. However, clinical symptoms of respiratory infection such as fever or hypothermia, cough, dyspnea, myalgia, arthralgia, headache and delirium are unspecific. Radiological examination and microbiological tests are mandatory to establish the diagnosis of (bacterial) pneumonia. But, diagnostic properties especially radiological examinations are limited in immobilized stroke patients. Thus the prompt identification of infections in stoke patients can be clinically challenging and the diagnostic gold-standard algorithm is time-consuming and may delay early antibiotic therapy⁸. The idea to instead routinely perform microbiological assessment on admission even in severely affected stroke patients has not shown any benefit since early bacteremia seems not to be common despite the high rate of downstream pneumonia in these patients¹². The approach of preventive antibiotic treatment in all stroke patients has shown no significant benefit concerning functional outcome or mortality¹³ moreover this approach will on the long run introduce problems with antimicrobial resistances.

Thus the identification of vulnerable patients that really benefit from early antibiotic treatments is crucial.

The use of blood biomarkers to identify patients, vulnerable for post-stroke infections, may help to implement treatment more rapidly. Currently, the most frequently studied marker is C-reactive protein. Although it is an established inflammatory marker in clinical routine, it has only a moderate predictive value for post-stroke infections⁸. Inflammatory markers (such as CRP, white blood count (WBC), etc.), as well as clinical and radiological signs, unfortunately occur often after the onset of infection with a consecutive delay in early antibiotic treatment. Moreover most of these makers are not specific to bacterial infections, they rather reflect an overall systemic inflammatory response. Therefore the identification of novel blood biomarkers to accurately predict post stroke infections, to choose the optimal prevention and or treatment strategy, is critical. "Omics" analysis is performed in the context of functional modules and networks that control and relay biological information. The widespread use of these enabling strategies and their applications to stroke sciences is set to have a huge impact on the understanding of post stroke infection progression as well as the discovery of markers to identify patients at highest risk for infection.

### Methods

aSAH patients included in the present study were hospitalized at the Pitié-Salpetriere hospital of Paris (France) between July 2004 and April 2008. aSAH was confirmed angiographically and by cerebral computed tomography (CT). The study was approved by the local ethical committee (Comité de Protection des Personnes, Pitié-Salpetriere, Paris, France). All patients or surrogates signed a written informed consent. The severity of patients' state was established on admission at the intensive care unit (ICU) using the World Federation of Neurosurgical Societies (WFNS). Depending on the location and the size of the aneurysm, patients were untreated, treated by surgical clipping or treated by endovascular coiling. More details on the clinical monitoring and treatment have been previously described by Turck *et al.* (Turck N, Vutskits L, Sanchez-Pena P, Robin X, Hainard A, Gex-Fabry M, Fouda C, Bassem H, Mueller M, Lisacek F, Puybasset L, Sanchez J-C, (2010) A multiparameter panel method for outcome prediction following aneurysmal subarachnoid hemorrhage. Intensive Care Med 36: 107-115). Among the 198 consecutive hospitalized patients, 137 were excluded from the present study due to: 1) presence of more than one haemorrhagic event, 2) admission at the hospital more than 48 hours after the onset of the symptoms, 3) missing clinical information or 4) insufficient sample volume to perform our assays. Sixty-one patients were then finally included in this study. Plasma samples were collected the day of arrival at the hospital and subsequently every day for 10 consecutive days (D1 to D10). The infection status was define every day based on the criteria established by "The International Sepsis Forum Consensus Conference on Definitions of Infection in the Intensive Care Unit (Calandra T, Cohen J, (2005) The international sepsis forum consensus conference on definitions of infection in the intensive care unit. Crit Care Med 33: 1538-1548). When a suspicion of infection with systematic clinical and biological criteria was obtained, bacteriological samples were taken. Antibiotic therapy was started as soon as the suspicion of the infection appeared and readjusted once the results of the cultures were obtained.

The blood targets content of patients presenting or not infection were quantified and compared using ELISA sandwiches. ELISAs were performed using Neopterin from Brahms (Thermo), Troponin I from Roche, CRP from millipore, SAA, IP-10, IFN-γ, IL-1β, IL-10, IL-12 p70, IL-13, IL-2, IL-4, IL-6, IL-8 and TNF-α from Mesoscale, IL-29 from Sigma and S100b from Abnova. Each plasma sample were assayed in duplicate and distributed randomly on the plates.

Protein levels were initially expressed in relative optical or fluorescence unit (RFU) and concentrations were calculated using a calibration curve obtained on the same plate with the recombinant proteins. Statistical analyses were performed using IBM SPSS Statistics software version 19.0.0 (IBM Corporation, NY, USA). To assess the ability of proteins to discriminate between different populations, non-parametric tests were performed. A Wilcoxon matched pairs test was performed for age and sex matched data and a Mann-Whitney for non-matched data. For data containing more than two groups, a one-way ANOVA Kruskal-Wallis test was used. Receiver Operating Characteristic (ROC) curves analysis was performed and cut-off (CO) points obtained from the curves. Optimal threshold values were chosen to provide the highest specificity for 100% sensitivity or the highest sensitivity for 100% specificity.

One goal that was achieved was to provide a panel (i.e. a small set of two or more) of biomolecules that can be useful in a clinical setting. Panels of molecules can provide better diagnostic value than single biomarkers. The idea is that each member of the panel provides a different angle to the diagnosis and taken together they lead to a more accurate prediction. Each member of the panel must fulfil several criteria: firstly it must have a predictive power itself, i.e. it must be able to distinguish the disease types to a certain extent. Secondly it must be easy to measure with high reproducibility. Thirdly, it should have a central role in the biological processes that were found by the network analysis.

Neopterin, Troponin I, CRP, SAA, IP-10, IFN-γ, IL-1β, IL-10, IL-12 p70, IL-13, IL-2, IL-4, IL-6, IL-8, TNF-α, IL-29, S100b and white blood cell count were considered for panel analysis as well as clinical information such as age, GCS, WFNS and Fisher. We developed PanelomiX toolbox, which is able to extract optimal panels from a small number of molecules and provides a simple, easy to interpret set of threshold rules for disease type classification. The rule-based classifier just counts the number of molecules whose quantity passes specific threshold values. It mimics the way many clinical scores are built and is therefore easy to understand by people working in a clinical environment. Briefly, the optimized cutoff values were obtained by iterative permutation-response calculations using all available parameters. Each cutoff value was changed iteratively by quantities of 2% increment, and specificity was determined after each iteration until a maximum of specificity was achieved for 100% sensitivity.

### Results

Table 1 shows the performance of the different parameters as a single variable to detect infected and non-patients with SAA as the best blood molecule with an AUC of 75% (95% SE - 40% SP).

**Table 1:**

| Day | Molecule | Accuracy | | | 100% SP | | 100% SE | |
|---|---|---|---|---|---|---|---|---|
| | | AUC | SP | SE | SP | SE | SP | SE |
| D1 | NP | 58.6 | 66.7 | 53.8 | 96.3 | 13.5 | 22.2 | 90.4 |
| | S100b | 70.5 | 65.2 | 72.5 | 91.3 | 33.3 | 17.4 | 92.2 |
| | SAA | 75.3 | 80 | 71.8 | 95 | 33.3 | 40 | 94.8 |
| | CRP | 70.9 | 50 | 84.6 | 90 | 41 | 30 | 92.3 |
| | WBC | 76.3 | 81.8 | 72.2 | 90.9 | 58.3 | 18.1 | 97.2 |
| | WFNS | 79.1 | 92.6 | 51.9 | 100 | 31.5 | 0 | 100 |
| | Fisher | 81.5 | 70.4 | 81.5 | 100 | 18.5 | 37 | 98.1 |
| | Glasgow | 78.8 | 92.6 | 50 | 96.3 | 40.7 | 0 | 100 |
| | Age | 46.5 | 44.4 | 74.1 | 100 | 1.9 | 3.7 | 100 |

Table 2 shows the performance of the best combination of two parameters with an AUC of 80% (100% SE - 61% SP).

**Table 2**

| | | | | | |
|---|---|---|---|---|---|
| Best combination of 2 molecules (SAA fix) | | | | | |
| Test markers: NP, S100B, CRP, WBC, SAA, WFNS, Fisher, Glasgow, Age | | | | | |
| | | | | | |

| | Best accuracy | | | ↑ SP | ↑ SE |
|---|---|---|---|---|---|
| | AUC | SP | SE | | |
| WFNS, SAA | 80.4 | 100 | 60.9 | - | 100% SE, 60.9 SP |
| NP-SAA | - | - | - | 100% SP, 33.3% SE | - |

Table 3 shows the performance of the best combination of three parameters with an AUC of 89% (100% SE - 78% SP).

**Table 3**

| | | | | | |
|---|---|---|---|---|---|
| Best combination of 3 molecules (SAA fix) | | | | | |
| Test markers: NP, S100B, CRP, WBC, SAA, WFNS, Fisher, Glasgow, Age | | | | | |
| | | | | | |

| | Best accuracy | | | ↑ SP | ↑ SE |
|---|---|---|---|---|---|
| | AUC | SP | SE | | |
| NP+SAA+WFNS | 89.1 | 100 | 78.3 | 100% SP, 78.3% SE | - |
| WBC, Age, SAA | - | - | - | - | 100% SE, 78.3% SP |

Table 4 shows the performance of the best combination of four parameters with an AUC of 94% (100% SE - 83% SP).

**Table 4**

| | Best accuracy | | | ↑ SP | ↑ SE |
|---|---|---|---|---|---|
| | AUC | SP | SE | | |
| NP+S100B+Troponin+SAA | 94.4 | 88.9 | 95.7 | 95.7% SP, 88.9% SE | |
| NP+S100B+WBC+SAA | | | | | 100% SE, 82.6% SP |
| NP+Troponin+WBC+SAA | | | | | |
| NP+WBC+SAA+Age | | | | | |
| NP+WFNS+WBC+SAA | | | | | |
| NP+WFNS+Age+SAA | | | | | |
| SAA+WBC+WFNS+Age | | | | | |

Improved specificity (>83%) for 100% sensitivity to rule out non infected patients.

This results and the current disclosure has the potential to make a world broad impact on the clinical practice in the management of post-stroke/SAH infection. The current results have lead to a predictive panel of markers with very high value. The results described herein on SAA, CRP and Neopterin panels of at least two of these molecules highlight that a POCT or an array can be readily used with known methods and devices. The results as disclosed herein has the potential to make a world broad impact on the clinical practice in the management of acute post brain infection. Accurate prediction of the infection after brain injury at admission or already several days before the infection development, making possible an earlier antibiotic therapy and a better management of the patients is crucial. The current disclosure contributes thus to a significant improvement in patient care and handling.

### Example 2

### Baseline blood biomarkers to predict post-stroke infections

### Cohort description

Seventy-eight acute stroke patients were included in whom blood biomarkers were measured in samples obtained within 6 hours of stroke onset, in fact the median time of extraction was 2.3 hours (1.48-3.57) after stroke onset.

Regarding infections appearance: 27 patients did not have any infection (34.6%), 27 had pneumonia (34.6%), 13 (16.7%) had bronchitis and 11 (14.1%) had urinary infection.

Regarding demographic data: The median age of all patients was 81 (74-86), 33 patients (42.3%) were male, two patients (2.9%) took alcohol and eight (11.4%) smoked. 53 (67.9%) had hypertension, 36 (46.2%) had dislipemia and 20 (25.6%) had diabetes. 12 patients (15.4%) had coronary disease, 27 (37.5%) had atrial fibrillation and only five (6.4%) had peripheral arterial disease.

Regarding stroke characteristics at emergency department arrival: median NIHSS at baseline was 17 (9-20), mean SBP was 153.87±28.9, median DBP was 80 (74-90) and median glycaemia was 124 (103-162.5). Regarding stroke onset, 12 patients had wake-up stroke (15.4%). Regarding stroke etiology 38 (50%) patients were cardioembolic, 12 (15.8%) were atherotrombotic, 22 (28.9%) were undetermined and only 1 was a lacunar stroke. Reagrding OCSP classification on stroke extension, 53 (69.7%) patients were TACI, 18 (23.7%) were PACI, 4 (5.3%) were POCI and only 1 was LACI. 47 (60.3%) received treatment at acute phase and 33 (70.2%) from the ones that received any treatment at acute phase were thrombolysis.

**Biomarkers of interest were measured in plasma (EDTA):** sTREM-1 Quantiquine R&D [reference DTRM10C]; IL10 HSensitive Luminex R&D [reference LHSCM000 & LHSCM217]; pro-ADM BRAHMS-Kryptor [reference 829.050]; SAA, Human, ELISA kit Hycultk [reference HK333-02] and D-Dimer Stago [reference 947].

**Other Biomarkers of interest were measured in serum:** IL29 Cusabio [reference CSB-E14290h]; presepsin (sCD14)-CRP-IFFBP3 3-plex Luminex R&D [reference custom LXSAHM]; IL13 HSensitive Luminex R&D [reference LBHS000 and LBHS213]; suPAR Quantiquine R&D [reference DPU00] and Neopterin BRAHMS-EIA [reference 99.1].

In the next sections we compare the differences among the measured biomarkers in the four groups of patients having "no infection", "respiratory infection (pneumonia)", "respiratory infection (bronchitis)" and "urinary infections".

### A. Comparison between stroke patients that become infected or not (Any type of infection)

### Biomarkers

Levels of IL10 and Proadrenomedullin (ProADM) had a trend to be higher in infected patients (p=0.222 and p=0.134). We obtained the best cutoffs for both molecules: ProADM cut-off of 0.939 (sen=51%, sp=74.1%) and IL10 cut-off of 0.19 (sen=51%, sp=70.4%). Patients that had levels of ProADM higher than 0.939 were more likely to have an infection (p=0.033), and patients with IL10 higher than 0.19 at Emergency Department arrival had a trend to have more infections in the follow-up (p=0.072).

### Univariate analysis

Patients that did not have wake-up stroke or had TACI or POCI were more likely to have an infection (p=0.019 and p=0.028). Male patients had a trend to have infection (p=0.099). NIHSS at baseline was slightly higher among the ones with infections [16 (9.5-18) vs 17 (9-21), p= 0.181].

### Basic Logistic regression

Independent predictors of infection were: proadrenomedullin 0.939 [OR 4.70 (1.36-16.16), p=0.014], sex [OR 0.26 (0.08-0.82), p=0.022] and NIHSS [OR 1.095 (1.005-1.195), p=0.039],
Logistic regression including significant variables at the univariate analysis Independent predictors of infection were: Wake-up strokes [OR 0.05 (0.008-0.32), p=0.002], OCSP [OR 3.67 (1.22-10.99), p=0.02], proadrenomedullin 0.939 [OR 6.32 (1.62-24.65), p=0.008],

### B. Comparison between stroke patients that had a RESPIRATORY INFECTION (Pneumonia+bronchitis) vs those with no infection or urinary infections

### Biomarkers

Patients that had respiratory infection, had higher levels of ProADM (p=0.05). Also levels of IL10, Neopterin and SAA had a trend to be higher in patients with respiratory infection (p=0.156, p=0.271 and p=0.28). Best cut-offs were obtained: ProADM cut-off of 0.9703 (sen=55%, sp=73.7%), IL10 cut-off of 0.19 (sen=55%, sp=64.9%) and SAA cut-off of 2980.4 (sen=60%, sp=60.5%). Patients that had levels of ProADM higher than 0.9703, had IL10 higher than 0.19 or had with SAA higher than 2980.4 were more likely to have an infection (p=0.032, p=0.01 and p=0.041, respectively).

### Univariate analysis

Patients that did not have wake-up stroke, had coronary artery disease or had higher levels of glycaemia were more likely to have an infection (p=0.009, p=0.016 and p=0.039). Patients that had diabetes had a trend to have a respiratory infection (p=0.052).

### Basic Logistic regression

Independent predictors of respiratory infection were: proadrenomedullin 0.97 [OR 3.83 (1.25-11.78), p=0.019], with trends for sex and NIHSS. Logistic regression including significant variables at the univariate analysis Independent predictors of respiratory infection were:
Wake-up strokes [OR 0.092 (0.015-0.54), p=0.009], glycemia [OR 1.014 (1.00-1.027), p=0.048], and proadrenomedullin 0.97 [OR 4.096 (1.33-12.54), p=0.014].

### C. Comparison between stroke patients that developed URINARY INFECTION vs those without infections or who developed other type of infections

### Biomarkers

Patients with urinary infection had a trend to have lower levels of CD14 (p=0.256) and higher levels of IGFBP3 (p=0.285). Best cut-offs were obtained: IGFBP3 cut-off of 2301.5 (sen=83.3%, sp=50%). Patients that had levels of IGFBP3 higher than 2301.5 were more likely to have an urinary infection (p=0.033).

### Univariate analysis

Patients that had lower SBP and had lower levels of glycaemia were more likely to have urinary infection (p=0.009 and p=0.048).

### Biomarker Logistic regression

The only independent predictor of urinary infection was IGFBP3 (2301.5) [OR 5 (1.017-24.59), p=0.048].

### D. Comparison between stroke patients that developed PNEUMONIA vs those with NO INFECTION

### Biomarkers

Patients with pneumonia had a trend to have higher levels of IL10 and ProADM (p=0.126 and p=0.135). Best cut-offs were obtained: ProADM cut-off of 1.008 (sen=44.4%, sp=81.5%). Patients that had levels of ProADM higher than 1.008 were more likely to have pneumonia (p=0.04).

### Univariate analysis

Patients that had wake-up stroke were more likely to have pneumonia (p=0.004). Also, male patients, the ones that had TACI or POCI strokes had a trend to have pneumonia (p=0.054, and p=0.053).

Logistic regression including significant variables at the univariate analysis Independent predictors of pneumonia were: OCSP [OR 7.15 (1.40-36.32), p=0.018] and ProADM (> 1.008) [OR 11.27 (1.61-78.64), p=0.015]. In this case the combination of both variables gave a good Area Under the Curve (AUC =0.84).

### E. Comparison between stroke patients that developed Bronchitis vs those with NO INFECTION

### Biomarkers

Patients that had higher levels of ProADM had a trend to have bronchitis (p=0.1). Best cut-offs were obtained: ProADM cut-off of 0.946 (sen=69.2%, sp=74.1%). Patients that had levels of ProADM higher than 0.946 were more likely to have bronchitis (p=0.009).

### Univariate analysis

Patients that had diabetes and peripheral artery disease were more likely to have bronchitis (p=0.032 and p=0.029).

Logistic regression including significant variables at the univariate analysis Independent predictors of bronchitis were: ProADM (> 0.946) [OR 11.42 (1.94-67.26), p=0.007].

### F. Comparison between stroke patients that developed any RESPIRATORY INFECTION (Pneumonia+bronchitis) vs those without any infection

### Biomarkers

Patients that had respiratory infection, had a trend to have higher levels of ProADM and IL10 (p=0.066 and p=0.167). Best cut-offs were obtained: ProADM cut-off of 0.946 (sen=55%, sp=74.1%). Patients that had levels of ProADM higher than 0.946, were more likely to have a respiratory infection (p=0.018).

### Univariate analysis

Patients that did not have wake-up stroke were more likely to have a respiratory infection (p=0.011). Patients that had TACI or POCI strokes had a trend to have a respiratory infection (p=0.055).

### Basic Logistic regression

Independent predictors of respiratory infection were: proadrenomedullin (>0.946) [OR 5.12 (1.39-18.88), p=0.014] and sex [OR 0.29 (0.093-0.95).

Logistic regression including significant variables at the univariate analysis Independent predictors of respiratory infection were: Wake-up strokes [OR 0.081 (0.013-0.49), p=0.007] and proadrenomedullin (>0.946) [OR 5.007 (1.45-17.27), p=0.011].

### Example 3

### Post-stroke infections are related with IL-29 level.

IL29 was measured in 16 stroke patients at patients arrival to the emergency department (<4.5h) using the Cusabio ELISA kit (pg/mL). The mean value of IL29 at baseline was 8.017 and at 24 hours 32.42. The mean age of these 16 patients was 73.75±16.19. A half of patients were females, also a half had hypertension or atrial fibrillation, 37.5% had diabetes, 25% had dyslipidemia, 7 patients (43.8%) had coronary artery disease, only 3 people smoke (18.8%) and one had previous stroke.

Ten patients (62.5%) were cardioembolic, one was atherothrombotic and 5 (31.3%) were undetermined. Half of patients improved at 24 hours, the 53.3% improved at 48 hours and at discharge decreased to 46.7%. The median mRS at 3^{rd} month was 2 (0-5), there were two deaths at 3^{rd} month (12.5%) and 7 patients were dependent at 3^{rd} month (43.8%). Two patients (12.5%) had a severe infection and 4 (26.7%) had mild infection or respiratory infection. In total there were 6 infections (40%).

**IL29 values at baseline**

| | No | Yes | p-value |
|---|---|---|---|
| Gender (female) | 0.197 (0.197-1.431) | 6.82 (0.2-22.35) | 0.05 |
| Smoke | 2.664 (0.197-2.664) | 0.197 (0.197-0.197) | 0.111 |
| Hypertension | 0.197 (0.197-3.846) | 2.597 (0.197-21.035) | 0.328 |
| Diabetes | 0.2 (0.197-8.646) | 1.431 (0.197-5.028) | 0.958 |
| Dyslipidemia | 1.433 (0.197-9.961) | 0.197 (0.197-2.613) | 0.316 |
| Atrial fibrilation | 0.197 (0.197-3.846) | 2.598 (0.197-22.35) | 0.279 |
| Ischemic cardiopathy | 4.994 (0.197-8.646) | 0.197 (0.197-1.431) | 0.252 |
| Hemorrhagic transformation | 0.197 (0.197-6.837) | 1.433 (0.2-18.044) | 0.599 |
| TOAST | | | 0.462 |
| Cardioembolic | ---- | 0.2 (0.197-11.276) | |
| Undetermined | ---- | 0.197 (0.197-2.664) | |
| Mortality at 3^{rd} month | 0.197 (0.197-5.028) | 32.728 (4.994-60.462) | 0.15 |
| Dependent | 0.197 (0.197-3.846) | 0.202 (0.197-19.209) | 0.463 |
| Improvement 24 hours | 0.2 (0.197-6.837) | 1.431 (0.197-8.135) | 1 |
| Improvement 48 hours | 4.994 (0.2-6.837) | 0.197 (0.197-6.97) | 0.463 |
| Improvement at discharge | 1.433 (0.197-5.011) | 0.197 (0.197-5.737) | 0.613 |
| Mild infection | 2.664 (0.197-8.152) | 0.197 (0.197-4.422) | 0.343 |
| Severe infection | 0.197 (0.197-5.028) | 18.044 (2.664-33.424) | 0.305 |
| Respiratory infection | 0.202 (0.197-6.837) | 1.431 (0.197-18.044) | 0.949 |
| Infection | 0.202 (0.197-5.028) | 1.431 (0.197-8.646) | 0.955 |
| Age | R=0.158 | | 0.559 |
| NIHSS at baseline | R=0.471 | | 0.066 |
| NIHSS at 24 hours | R=0.465 | | 0.069 |
| NIHSS at 48 hours | R=0.578 | | 0.024 |
| NIHSS at discharge | R=0.475 | | 0.074 |

**IL29 values at 24 hours**

| | No | Yes | p-value |
|---|---|---|---|
| Gender (female) | 0.55 (0.197-15.228) | 30.158 (6.641-79.864) | 0.053 |
| Smoke | 25.578 (3.543-38.119) | 0.55 (0.374-2.714) | 0.126 |
| Hypertension | 1.338 (0.374-15.228) | 30.158 (6.641-79.864) | 0.128 |
| Diabetes | 15.663 (3.108-79.864) | 4.042 (0.197-30.158) | 0.414 |
| Dislipemia | 27.868 (5.748-39.414) | 0.374 (0.197-2.714) | 0.024 |
| Atrial fibrilation | 2.714(0.197-23.474) | 27.868 (5.748-36.824) | 0.181 |
| Ischemic cardiopathy | 3.543 (0.374-76.531) | 16.556 (4.878-36.824) | 0.755 |
| Hemorrhagic transformation | 3.108 (0.197-25.578) | 33.491 (17.953-38.119) | 0.188 |
| TOAST | | | 0.235 |
| Cardioembolic | ---- | 15.663 (3.108-33.491) | |
| Undetermined | ---- | 0.55 (0.197-7.534) | |
| Dependent | 3.108 (0.197-16.556) | 30.158 (5.748-36.824) | 0.171 |
| Improvement 24 hours | 5.748 (0.374-33.491) | 7.534 (3.108-32.496) | 0.71 |
| Improvement 48 hours | 30.158 (2.973-79.864) | 6.206(1.053-25.578) | 0.534 |
| Improvement at discharge | 21.286 (0.374-81.159) | 4.878 (1.338-7.534) | 0.524 |
| Mild infection | 5.313 (0.197-30.158) | 36.824 (18.687-79.864) | 0.469 |
| Severe infection | 4.878 (0.374-31.201) | 34.486 (30.158-39.414) | 0.308 |
| Respiratory infection | 3.108 (0.197-25.587) | 36.824 (33.491-38.119) | 0.161 |
| Infection | 3.108 (0.197-15.663) | 36.824 (30.158-39.414) | 0.127 |
| Age | R=0.393 | | 0.164 |
| NIHSS at baseline | R=0.693 | | 0.006 |
| NIHSS at 24 hours | R=0.593 | | 0.029 |
| NIHSS at 48 hours | R=0.634 | | 0.02 |
| NIHSS at discharge | R=0.565 | | 0.044 |

IL29 cut-off of 27.868 (sensitivity=100%, specificity=83.3%) for respiratory infection was done using ROC curves (p=0.022). The same cut-off was done for infection yes-no (sensitivity =66.7%, specificity =88.9%) (p=0.089).

### Example 4

### PATIENTS

We included 8 stroke patients not treated with tPA, with baseline blood samples collected within the first 12 hours after stroke onset and a serial blood sample profile at baseline, 24 hours, 48 hours, 72 hours, and a final blood sample collected from 5^{th} to 7^{th} day after stroke. The following biomarkers were measured: SAA, MR-proADM, UPAR and IL13.

Four patients developed infections during the first week of hospital admission. Of them, two had fever of unknown focus, and two had stroke-associated pneumonia (one of them had also a concomitant urinary infection). One of the pneumonias fulfilled classical CDC criteria (with radiological confirmation) while the other fulfilled the modified CDC criteria (probable SAP).

There were no differences on baseline variables between those patients with infections and those without infections.

| | | **ALL (N=8)** | **Infections (N=4)** | **No infections (N=4)** | **p** |
|---|---|---|---|---|---|
| **Age** | | 87.7 (86.3-92.3) | 89.8 (86.5-95) | 87.6 (84-90.1) | 0.343 |
| **Gender (male)** | | 3 (37.5%) | 1 (25%) | 2 (50%) | 0.999 |
| **Hypertension** | | 7 (87.5%) | 3 (75%) | 4 (100%) | 0.999 |
| **Diabetes** | | 1 (12.5%) | - | 1 (25%) | 0.429 |
| **Dyslipidemia** | | 2 (25%) | - | 2 (50%) | 0.999 |
| **Atrial fibrillation** | | 3 (37.5%) | 2 (50%) | 1 (25%) | 0.999 |
| **Baseline NIHSS** | | 14.5 (7-18) | 15.5 (11-18) | 10.5 (6.5-17.5) | 0.486 |
| **TOAST** | **LAA** | - | - | - | 0.999 |
| | **CE** | 5 (62.5%) | 3 (75%) | 2 (50%) | |
| | **LAC** | - | - | - | |
| | **UND** | 3 (37.5%) | 1 (25%) | 2 (50%) | |

The results are also depicted in the Figures.

There was no differences in temporal profile in the group of infections (p=0.663) but there were differences in in the group of no infections (p=0.024).

Analyzing SAA by time, there were differences between infected and no infected patients at baseline (p=0.029) and there was a trend at time 5 (p=0.057).

### Example 5

### Post stroke infection prediction by SAA

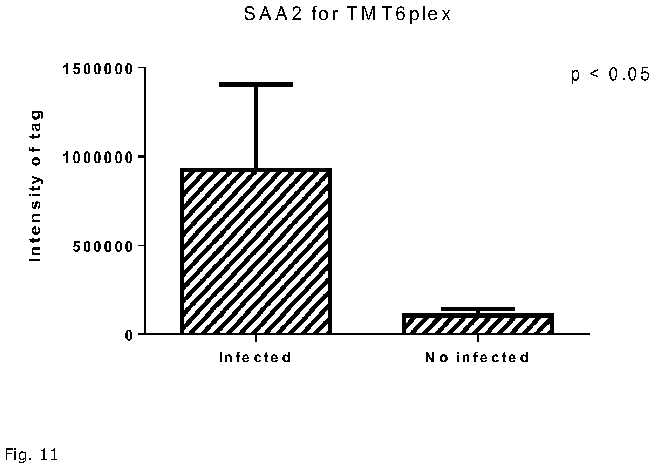

Proteomics preliminary results : a first study of infected ischaemic-stroke depleted plasma samples was performed to evaluate the feasibility of discovering potential biomarkers for infection prediction in stroke patients. The plasma proteomes of infected (n = 3) and non-infected (n = 3) stroke patients at hospital admission were compared using 6-plex TMT isobaric labelling quantitative MS. Proteomics revealed 17 significantly regulated proteins (p < 0.01). One of these, serum amyloid A (SAA), is shown in Fig. 11 below.

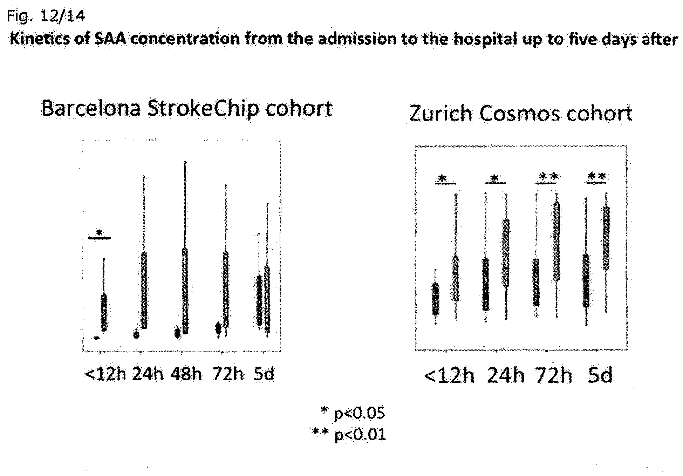

Verification preliminary results : The SAA result discovered using proteomics was verified further using an enzyme-linked immunosorbent assay (ELISA from MSD) in two, small, independent cohorts of stroke patients from Barcelona (StrokeChip, n = 8) and Zurich (COSMOS, n = 44). SAA was measured at five time points (< 12 h, 24 h, 48 h, 72 h and 5 days) in the StrokeChip cohort and four time points in the COSMOS cohort (< 12h, 24 h, 72 h and 5 days). ELISA revealed that at hospital admission, concentrations of SAA were already significantly higher in patients who were to develop an infection during hospitalisation (p < 0.01) in both the StrokeChip cohort (Fig. 12 left panel) and the COSMOS cohort (Fig. 12 right panel).

Post stroke infection prediction by MCP-3, CD244, Il-7, OSM, TRAIL, IL-6, SIRT2, Fit3L and CD6

### Preliminary results :

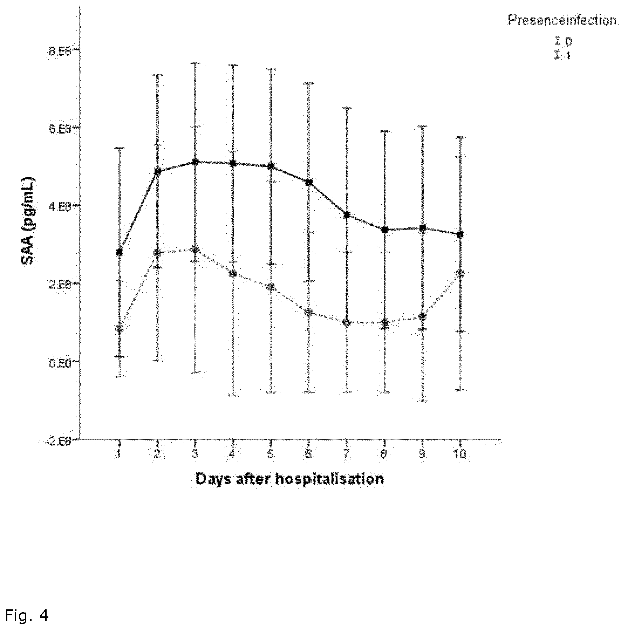

Several additional differentially expressed proteins were also verified using an immunoassay (Olink) on a limited number of stroke patients at hospital admission (non-infected, n = 3; infected, n = 3). Fig. 4, below, shows the most promising over- and under-expressed proteins, namely MCP-3, CD244, Il-7, OSM, TRAIL, IL-6, SIRT2, Fit3L and CD6.

The results are also depicted in Figure 15.

### Additional data:

**TABLE 5 Baseline Characteristics of All Patients, Stratified by infectios event^{a}**

| | **All Patients (*n* = 283)** | | **No infect (*n* = 224)** | | **infect (*n* = 59)** | | ***P* Value Logistic reg (OR)** |
|---|---|---|---|---|---|---|---|
| **Demographic data** | | | | | | | |
| Age, median (IQR), y | 75.3 | 62.5-82 | 75 | 61-81 | 78 | 68-83 | 0.08 |
| Women, *n* (%) | 115 | 40.64 | 82 | 36.6 | 33 | 55.9 | <0.008 |

| **Medical history, *n* (%)** | | | | | | | |
|---|---|---|---|---|---|---|---|
| Hypertension | 213 | 75.3 | 160 | 71.4 | 53 | 89.8 | 0.04 |
| Atrial fibrillation | 57 | 20.1 | 38 | 17 | 19 | 32.2 | 0.02 |
| Current smoking | 104 | 37.8 | 81 | 36.2 | 23 | 38.9 | 0.9 |
| Diabetes mellitus | 54 | 19.1 | 42 | 18.8 | 12 | 20.3 | 0.9 |
| Coronary heart disease | 61 | 21.6 | 50 | 22.3 | 11 | 18.6 | 0.49 |
| Dyslipidemia | 73 | 25.8 | 59 | 26.3 | 14 | 23.7 | 0.9 |
| Positive FA | 80 | 28.3 | 64 | 28.6 | 16 | 27.1 | 0.75 |
| Previous cerebrovascular event¹⁾ | 67 | 23.7 | 54 | 24.1 | 13 | 22.0 | 0.7 |
| PAVK | 25 | 8.8 | 20 | 8.9 | 5 | 8.5 | 0.92 |
| Modified Charlson Index | 1 | 0-2 | 1 | 0-2 | 1 | 0-2 | 0.1 |

| **Clinical data, median (IQR)** | | | | | | | |
|---|---|---|---|---|---|---|---|
| NIHSS at admission (points) | 5 | 2-10 | 4 | 2-8 | 10 | 5-17 | <0.001 |

| **Laboratory values, median (IQR)** | | | | | | | |
|---|---|---|---|---|---|---|---|
| WBC | 8.15 | 6.6-9.9 | 8 | 6.4-9.4 | 9.7 | 7.5-11.2 2 | <0.001 |
| Monocytes | 0.38 | 0.3-0.5 | 0.38 | 0.3-0.49 | 0.41 | 0.32-0.54 54 | 0.04 |
| CRP (mg/l) | 3.1 | 3-7.9 | 3 | 3-6 | 5.3 | 3-17 | 0.002 |
| PCT | 0.017 | 0.012-0.025 | 0.017 | 0.012-0.024 | 0.02 | 0.014-0.032 | 0.003 |
| Copeptine | 9.7 | 5-25.6 | 8.8 | 4.7-20.1 | 22.5 | 7.5-62.3 | <0.001 |
| MR-proANP(pmol/ | 138 | 78.8-232 | 122 | 73.6-215 | 213 | 136-326 | <0.001 |
| SAA_all_day0 | 6.2 | 2.7-20.75 | 5 | 2.5-12.7 | 16.7 | 4.7-73.3 | <0.001 |
| Kreatinin (mL/min) | 73.5 | 62-87 | 74 | 63-87 | 70 | 61-89 | 0.7 |
| Temp | 37 | 36.6-37.4 | 37 | 36.6-37.5 | 37 | 36.5-37.3 | 0.8 |

| **Lesion size on MR, DWI^{b}** | | | | | | | |
|---|---|---|---|---|---|---|---|
| Small (1-10mm³) | 117 | 41.3 | 97 | 43.3 | 20 | 33.9 | 0.01 |
| Medium (10-100mm³) | 39 | 13.8 | 28 | 12.5 | 11 | 18.6 | 0.34 |
| Large (>100mm³) | 8 | 2.8 | 2 | 0.9 | 6 | 10.2 | 0.003 |

| **TOAST** | | | | | | | |
|---|---|---|---|---|---|---|---|
| Large vessel stroke | 54 | 19.1 | 44 | 19.6 | 10 | 17 | 0.6 |
| cardioembolic | 97 | 34.3 | 68 | 31 | 29 | 49.2 | 0.008 |
| microangipathic | 49 | 17.3 | 43 | 19.2 | 6 | 10.2 | 0.1 |
| other | 13 | 4.6 | 11 | 4.9 | 2 | 3.4 | 0.6 |
| unknown | 69 | 24.4 | 57 | 25.5 | 12 | 20.3 | |

| **Character** | | | | | | | |
|---|---|---|---|---|---|---|---|
| TACS | 27 | 9.5 | 16 | 7.1 | 11 | 18.6 | 0.01 |
| PACS | 123 | 43.5 | 94 | 42 | 29 | 49.2 | 0.4 |
| LACS | 62 | 21.9 | 56 | 25 | 6 | 10.2 | 0.02 |
| POCS | 69 | 24.4 | 56 | 25 | 13 | 22.0 | 0.6 |

| **Infection** | | | | | | | |
|---|---|---|---|---|---|---|---|
| Pneumonia | 20 | 7.1 | 0 | 0 | 20 | 33.9 | |
| Urinary tract | 21 | 7.4 | 00 | 0 | 21 | 35.6 | |
| Other | 19 | 6.7 | 00 | 0 | 19 | 32.2 | |

| | | | | | | | |
|---|---|---|---|---|---|---|---|
| • * significant in univariate logistic regression (mortality) p<0.05 • ** significant in univariate logistic regression (mortality) p<0.01 | | | | | | | |

**TABLE 6 ((Table 2)) Multivariate logistic Regression Analysis with selected Variables significant in Table 1 and univariate logistic analysis (p<0.001) for infection**

| **Predictors** | **OR** | **Infection 95% CI** | | | ***P*** |
|---|---|---|---|---|---|
| NIHSS | 1.07 | 1.01 | | 1.10 | 0.008 |
| WBC_dayo | 1.15 | 1.04 | - | 1.10 | 0.009 |
| Log_SAA_all_day0 | 1.31 | 1.07 | - | 1.59 | **0.008** |

**TABLE 7 Baseline Characteristics of All Patients, Stratified by functional outcome**

| | **Functional outcome (*n* = 244)** | | **Good outcome (150)** | | **Bad outcome (94)** | | **(OR) p** |
|---|---|---|---|---|---|---|---|
| log_SAA_all_day0 | 6.23 | 2.7-20.8 | 4.8 | 2.5-12.1 | 10.7 | 4.1-76 | <0.001 |

**TABLE 8 Baseline Characteristics of All Patients, Stratified by mortality**

| | **Mortality (*n =* 244)** | | **Alive (215)** | | **dead (29)** | | **HR (p)** |
|---|---|---|---|---|---|---|---|
| log_SAA_all_day0 | 6.2 | 2.7-20.8 | 5.8 | 2.6-18.2 | 10.6 | 4.8-70.6 | <0.008 |

### Abbreviations List

A2DS2: Age, Atrial fibrillation, Dysphagie, Sex, stroke severity
aSAH: aneurysmal Subarachnoid Haemorrhage
AUC: Area Under the ROC Curve
CI: Confidence Interval
CID: Collision Induced Dissociation
CRP: C-reactive Protein
CSF: Cerebrospinal Fluid
CT: Computed Tomography
ELISA: enzyme-linked immunosorbent assay
EVD: External Ventricular Drain
GCS: Glasgow Coma Scale
GOS: Glasgow Outcome Scale
GTP: Guanosin-5-Triphosphate
HCD: High Collision Dissociation
ICU: Intensive Care Unit
IPG: Immobilized pH Gradient
MSD: Mesoscale Discovery
ROC: Receiver Operating Characteristic
PCT: Procalcitonin
SE: Sensitivity
SMARTER: Simultaneous Marker discovery And verification for the Rapid Translation of Exogenous Reference material
SP: Specificity
TEAB: Tetraethylammonium bromide
TCEP: Tris-(2-carboxyethil) phosphine hydrochloride
TMT: Tandem Mass Tag
WB: Western Blot
WBC: White Blood Cells
WFNS: World Federation of Neurosurgical Societies

### Reference List

1. Suarez JI, Tarr RW, Selman WR, (2006) Aneurysmal Subarachnoid Hemorrhage. New England Journal of Medicine 354: 387-396
2. Linn FHH, Rinkel GJE, Algra A, van Gijn J, (1996) Incidence of Subarachnoid Hemorrhage: Role of Region, Year, and Rate of Computed Tomography: A Meta-Analysis. Stroke; a journal of cerebral circulation 27: 625-629
3. de Rooij NK, Linn FH, van der Plas JA, Algra A, Rinkel GJ, (2007) Incidence of subarachnoid haemorrhage: a systematic review with emphasis on region, age, gender and time trends. J Neurol Neurosurg Psychiatry 78: 1365-1372
4. Schievink WI, (1997) Intracranial Aneurysms. New England Journal of Medicine 336: 28-40
5. Dirnagl U, Klehmet J, Braun JS, Harms H, Meisel C, Ziemssen T, Prass K, Meisel A, (2007) Stroke-Induced Immunodepression: Experimental Evidence and Clinical Relevance. Stroke; a journal of cerebral circulation 38: 770-773
6. Offner H, Vandenbark AA, Hurn PD, (2009) Effect of experimental stroke on peripheral immunity: CNS ischemia induces profound immunosuppression. Neuroscience 158: 1098-1111
7. Haeusler KG, Schmidt WUH, Föhring F, Meisel C, Helms T, Jungehulsing GJ, Nolte CH, Schmolke K, Wegner B, Meisel A, Dirnagl U, Villringer A, Volk HD, (2008) Cellular Immunodepression Preceding Infectious Complications after Acute Ischemic Stroke in Humans. Cerebrovascular Diseases 25: 50-58
8. Sarrafzadeh A, Schlenk F, Meisel A, Dreier J, Vajkoczy P, Meisel C, (2011) Immunodepression after aneurysmal subarachnoid hemorrhage. Stroke; a journal of cerebral circulation 42: 53-58
9. Frontera JA, Fernandez A, Schmidt JM, Claassen J, Wartenberg KE, Badjatia N, Parra A, Connolly ES, Mayer SA, (2008) Impact of nosocomial infectious complications after subarachnoid hemorrhage. Neurosurgery 62: 80-87
10. Ulm L, Ohlraun S, Harms H, Hoffmann S, Klehmet J, Ebmeyer S, Hartmann O, Meisel C, Anker SD, Meisel A, (2013) STRoke Adverse outcome is associated WIth NoSocomial Infections (STRAWINSKI): procalcitonin ultrasensitive-guided antibacterial therapy in severe ischaemic stroke patients - rationale and protocol for a randomized controlled trial. International journal of stroke : official journal of the International Stroke Society 8: 598-603
11. Wartenberg KE, Stoll A, Funk A, Meyer A, Schmidt JM, Berrouschot J, (2011) Infection after acute ischemic stroke: risk factors, biomarkers, and outcome. Stroke research and treatment 2011: 830614
12. Kammersgaard LP, Jorgensen HS, Reith J, Nakayama H, Houth JG, Weber UJ, Pedersen PM, Olsen TS, (2001) Early infection and prognosis after acute stroke: The Copenhagen Stroke Study. Journal of Stroke and Cerebrovascular Diseases 10: 217-221
13. Leire Azurmendi VD, Natalia Tiberti, Natacha Kapandji, Paola Sanchez, Asita Sarrafzadeh, Louis Puybasset, Natacha Turck, Jean-Charles Sanchez, (2015) Neopterin plasma levels correlate with infection and long-term outcome in aneurysmal subarachnoid haemorrhage. Journal of Neurosurgery - [Epub ahead of print]
14. Oconnor E VB, Mashongonyika C, Lipman J, Hall J, Thomas P., (2004) Serum Procalcitonin and C-reactive proteins as markers of sepsis and outcome in patients with neurotrauma and subarachnoid haemorrhage. Anesth Intensive Care 32 465-470
16. Teasdale GM, Drake CG, Hunt W, Kassell N, Sano K, Pertuiset B, De Villiers JC, (1988) A universal subarachnoid hemorrhage scale: report of a committee of the World Federation of Neurosurgical Societies. Journal of Neurology, Neurosurgery, and Psychiatry 51: 1457
17. Teasdale G, Murray G, Parker L, Jennett B, (1979) Adding up the Glasgow Coma Score. Acta Neurochir Suppl 28: 13-16
18. Claassen J, Bernardini GL, Kreiter K, Bates J, Du YE, Copeland D, Connolly ES, Mayer SA, (2001) Effect of Cisternal and Ventricular Blood on Risk of Delayed Cerebral Ischemia After Subarachnoid Hemorrhage:: The Fisher Scale Revisited. Stroke; a journal of cerebral circulation 32: 2012-2020
19. Jennett B, Bond M, (1975) Assessment of outcome after severe brain damage. Lancet 1: 480-484
20. Calandra T, Cohen J, (2005) The international sepsis forum consensus conference on definitions of infection in the intensive care unit. Crit Care Med 33: 1538-1548
21. Dayon L, Turck N, Kienle S, Schulz-Knappe P, Hochstrasser DF, Scherl A, Sanchez JC, (2010) Isobaric tagging-based selection and quantitation of cerebrospinal fluid tryptic peptides with reporter calibration curves. Analytical chemistry 82: 848-858
22. Dayon L, Turck N, Scherl A, Hochstrasser DF, Burkhard PR, Sanchez J-C, (2010) From Relative to Absolute Quantification of Tryptic Peptides with Tandem Mass Tags: Application to Cerebrospinal Fluid. CHIMIA International Journal for Chemistry 64: 132-135
23. Dayon L, Pasquarello C, Hoogland C, Sanchez J-C, Scherl A, (2010) Combining low- and high-energy tandem mass spectra for optimized peptide quantification with isobaric tags. Journal of Proteomics 73: 769-777
24. Gluck F, Hoogland C, Antinori P, Robin X, Nikitin F, Zufferey A, Pasquarello C, Fétaud V, Dayon L, Müller M, Lisacek F, Geiser L, Hochstrasser D, Sanchez J-C, Scherl A, (2013) EasyProt - An easy-to-use graphical platform for proteomics data analysis. Journal of Proteomics 79: 146-160
25. Elias JE, Gygi SP, (2007) Target-decoy search strategy for increased confidence in large-scale protein identifications by mass spectrometry. Nat Methods 4: 207-214
26. Breitwieser FP, Muller A, Dayon L, Kocher T, Hainard A, Pichler P, Schmidt-Erfurth U, Superti-Furga G, Sanchez JC, Mechtler K, Bennett KL, Colinge J, (2011) General statistical modeling of data from protein relative expression isobaric tags. J Proteome Res 10: 2758-2766
27. Tiberti N, Hainard A, Lejon V, Robin X, Ngoyi DM, Turck N, Matovu E, Enyaru J, Ndung'u JM, Scherl A, Dayon L, Sanchez J-C, (2010) Discovery and Verification of Osteopontin and Beta-2-microglobulin as Promising Markers for Staging Human African Trypanosomiasis. Molecular & Cellular Proteomics : MCP 9: 2783-2795
28. Tan HT, Tan S, Lin Q, Lim TK, Hew CL, Chung MC, (2008) Quantitative and temporal proteome analysis of butyrate-treated colorectal cancer cells. Mol Cell Proteomics 7: 1174-1185
29. Salvisberg C, Tajouri N, Hainard A, Burkhard PR, Lalive PH, Turck N, (2014) Exploring the human tear fluid: discovery of new biomarkers in multiple sclerosis. Proteomics Clin Appl 8: 185-194
30. van Bussel BCT, Henry RMA, Schalkwijk CG, Ferreira I, Feskens EJM, Streppel MT, Smulders YM, Twisk JWR, Stehouwer CDA, (2011) Fish Consumption in Healthy Adults Is Associated with Decreased Circulating Biomarkers of Endothelial Dysfunction and Inflammation during a 6-Year Follow-Up. The Journal of Nutrition 141: 1719-1725
31. Robin X, Turck N, Hainard A, Tiberti N, Lisacek F, Sanchez JC, Muller M, (2011) pROC: an open-source package for R and S+ to analyze and compare ROC curves. BMC Bioinformatics 12: 1471-2105
32. M. LD, S. P, M. P, Diagnosis of bacterial infection in the ICU: General principles. Intensive Care Med 20: 0-0
33. Wartenberg KE, Stoll A, Funk A, Meyer A, Schmidt JM, Berrouschot J, (2011) Infection after Acute Ischemic Stroke: Risk Factors, Biomarkers, and Outcome. Stroke research and treatment 2011
34. Fluri F, Morgenthaler NG, Mueller B, Christ-Crain M, Katan M, (2012) Copeptin, Procalcitonin and Routine Inflammatory Markers-Predictors of Infection after Stroke. PloS one 7: e48309
35. Ruokonen E, Ilkka L, Niskanen M, Takala J, (2002) Procalcitonin and neopterin as indicators of infection in critically ill patients. Acta Anaesthesiol Scand 46: 398-404
36. Hug A, Murle B, Dalpke A, Zorn M, Liesz A, Veltkamp R, (2011) Usefulness of serum procalcitonin levels for the early diagnosis of stroke-associated respiratory tract infections. Neurocrit Care 14: 416-422
37. Malle E DBF, (1996) Human serum amyloid A (SAA) protein: a prominent acute-phase reactant for clinical practice. Eur J Clin Invest 26: 427-435
38. Gabay C, Kushner I, (1999) Acute-Phase Proteins and Other Systemic Responses to Inflammation. New England Journal of Medicine 340: 448-454
39. Nakayama T, Sonoda S, Urano T, Yamada T, Okada M, (1993) Monitoring both serum amyloid protein A and C-reactive protein as inflammatory markers in infectious diseases. Clinical Chemistry 39: 293-297
40. Cicarelli DD, Vieira JE, Bense, #xF1, or F, #xE1, Martins bE, (2008) Comparison of C-Reactive Protein and Serum Amyloid A Protein in Septic Shock Patients. Mediators of Inflammation 2008
41. Hultén C DS, (2002) Serum amyloid A (SAA) as an aid in the management of infectious disease in the foal: comparison with total leucocyte count, neutrophil count and fibrinogen. Equine Vet J 347: 693-698
42. Yuan H, Huang J, Lv B, Yan W, Hu G, Wang J, Shen B, (2013) Diagnosis Value of the Serum Amyloid A Test in Neonatal Sepsis: A Meta-Analysis. BioMed Research International 2013: 9
43. Arnon S LI, Regev R, Lis M, Shainkin-Kestenbaum R, Dolfin T, (2002) Serum amyloid A protein in the early detection of late-onset bacterial sepsis in preterm infants. J Perinat Med 30: 329-332
44. Stelmasiak JIaZ, (2000) PROGNOSTIC IMPORTANCE OF MONITORING SERUM AMYLOID A PROTEIN (SAA) IN PATIENTS WITH CEREBRAL INFARCTION. Acta clin Croat 39
45. Dirk Brämer HH, Albrecht Günther, Samuel Nowack, Frank M Brunkhorst, Otto W Witte and Dirk Hoyer, (2014) Study protocol: prediction of stroke associated infections by markers of autonomic control. BMC neurology 14
46. Emsley HC, Smith CJ, Tyrrell PJ, Hopkins SJ, (2008) Inflammation in acute ischemic stroke and its relevance to stroke critical care. Neurocrit Care 9: 125-138
47. F. WA, Infection control and prevention strategies in the ICU. Intensive Care Med 20: 0-0
48. Harms H, Prass K, Meisel C, Klehmet J, Rogge W, Drenckhahn C, Göhler J, Bereswill S, Göbel U, Wernecke KD, Wolf T, Arnold G, Halle E, Volk H-D, Dirnagl U, Meisel A, (2008) Preventive Antibacterial Therapy in Acute Ischemic Stroke: A Randomized Controlled Trial. PloS one 3: e2158
49. Schwarz S, Al-Shajlawi F, Sick C, Meairs S, Hennerici MG, (2008) Effects of Prophylactic Antibiotic Therapy With Mezlocillin Plus Sulbactam on the Incidence and Height of Fever After Severe Acute Ischemic Stroke: The Mannheim Infection in Stroke Study (MISS). Stroke; a journal of cerebral circulation 39: 1220-1227
50. Lampl Y BM, Gilad R, Lorberboym M, Dabby R, Rapoport A, Anca-Hershkowitz M, Sadeh M, (2007) Minocycline treatment in acute stroke: an open-label, evaluator-blinded study. Neurology 69: 1404-1410
51. Chaorro A, Horcajada JP, Obach V, Vargas M, Revilla M, Torres F, Cervera A, Planas AM, Mensa J, (2005) The Early Systemic Prophylaxis of Infection After Stroke Study: A Randomized Clinical Trial. Stroke; a journal of cerebral circulation 36: 1495-1500
52. Jin R, Yang G, Li G, (2010) Inflammatory mechanisms in ischemic stroke: role of inflammatory cells. Journal of Leukocyte Biology 87: 779-789

## Claims

1. Method for screening for a brain injury complications in a disease or disorder in a sample comprising the steps of using a sample under suitable conditions and detecting at least two biomarkers, preferably blood biomarkers, under suitable conditions wherein the biomarkers are selected from the group consisting of Serum Amyloid A (SAA), Neopterin, suPAR, CRP1, MCP-3, WBC, IL-10, IL-13, IL-29, pro-adrenomedullin, SAA1, NP1, IP-10, IFN-γ, copeptin, procalcitonin, IL-6, IL-8, TNF-α, IGFBP3, sTREM-1, D-Dimer, presepsin (sCD14), peroxiredoxin 1 to 6, CD244, Il-7, OSM, TRAIL, IL-6, SIRT2, Fit3L, S100b and CD6 or fragments, variants or mutants thereof.

2. Method according to claim 1 wherein the brain injury complication is selected from the group consisting of infection, e.g. pneumonia, urinary tract infection, haemorrhage, vasospasm and depression, and the disease or disorder is selected from the group consisting of stroke, traumatic brain injury (TBI), subarachnoid hemorrhage (SAH).

3. Method according to claim 1 or 2 wherein one or more biomarkers according to claim 1 is combined with a marker which is age, temperature, a defined GCS, GOS, NIHSS, Rankin and modified Rankin, Barthel Index, Fisher Score, or WFNS.

4. Method according to claim 1 or 2 wherein one or more biomarkers according to claim 1 is used for screening a defined age or age group, temperature or a defined GCS, GOS, NIHSS, A2DS2, Rankin and modified Rankin, Barthel Index, Fisher Score, or WFNS.

5. Method according to claim 3 or 4 wherein the age group has an age of less than 50, less than 60, or more than 60, more than 70, or more than 80.

6. Method according to any of claims 1 to 5 wherein the sample is blood, plasma, urine, saliva, tears (lachrymal fluid), or CSF.

7. Composition comprising or consisting of at least one or more markers useful for the detection of a brain injury complication of the group consisting of infection, e.g. pneumonia, urinary tract infection, depression, hemorrhage and vasospasm in a sample of an individual wherein the markers are selected from the group consisting of Serum Amyloid A (SAA), Neopterin, suPAR, CRP1, MCP-3, WBC, IL-10, IL-13, IL-29, pro-adrenomedullin, SAA1, NP1, IP-10, IFN-γ, copeptin, procalcitonin, IL-6, IL-8, TNF-α, IGFBP3, sTREM-1, D-Dimer, presepsin (sCD14), peroxiredoxin 1 to 6, CD244, Il-7, OSM, TRAIL, IL-6, SIRT2, Fit3L, S100b and CD6 or fragments, variants or mutants thereof.

8. Kit comprising or consisting of one or more markers according to claim 1.

9. Assay device comprising or consisting of one or more markers according to claim 1.

10. Assay device according to claim 9 which device comprises or consists of a biochip, biomarker panel, a carrier, or a test strip.

11. Method, composition, kit or assay according to any of the preceding claims wherein the sensitivity or specificity is more than 95%, preferably 100%, and/or the specificity or sensitivity is more than 50%, preferably more than 55%, more preferably more than 60%, and even more preferably more than 70%.

12. A method according to claim 1, which makes use of an algorithm.

13. A method for stratifying individuals for the treatment of brain injury complications using a method according to any of claims 1 to 6, a composition according to claim 7, a kit according to claim 8, an assay device according to claims 9 - 10 or/and an algorithm according to claim 12.

14. Method for the prediction of the susceptibility of an individual to develop a brain injury complication using a method according to any of claims 1 to 6, a composition according to claim 7, a kit according to claim 8, an assay device according to claims 9 - 10 or/and an algorithm according to claim 12.
